# EUROPEAN PATENT SPECIFICATION

(11) **EP 4 416 127 B1**
(45) Date of publication and mention of the grant of the patent: **11.03.2026**
(21) Application number: 22786050.9
(22) Date of filing: 12.10.2022
(51) Int. Cl.: C07C 67/14, C07C 69/716, C07C 67/283, C07C 51/09, C07C 59/19, C07B 59/00, C07F 7/18, A61K 49/10

(54) **PROCESS FOR PRODUCTION OF KETOCARBOXYLIC ACID VINYL ESTERS**
VERFAHREN ZUR HERSTELLUNG VON KETOCARBONSÄUREVINYLESTERN
PROCÉDÉ DE PRODUCTION D'ESTERS VINYLIQUES D'ACIDE CÉTOCARBOXYLIQUE

(30) Priority: 12.10.2021 EP 21202303
(43) Date of publication of application: 21.08.2024
(60) Divisional application: 26155096.6
(73) Proprietor: QuantView GmbH, 24118 Kiel (DE)
(72) Inventor: HERGES, Rainer, 24118 Kiel (DE); BRAHMS, Arne, 24118 Kiel (DE); PRAVDIVTSEV, Andrey, 24118 Kiel (DE); HÖVENER, Jan-Bernd, 24118 Kiel (DE)
(74) Representative: Taruttis, Stefan Georg
(86) International application number: PCT/EP2022/078451
(87) International publication number: WO 2023/062106

(56) References cited:
- WO-A1-2022/263619
- SALNIKOV OLEG G. ET AL: "Parahydrogen-Induced Polarization of 1- 13 C-Acetates and 1- 13 C-Pyruvates Using Sidearm Hydrogenation of Vinyl, Allyl, and Propargyl Esters", THE JOURNAL OF PHYSICAL CHEMISTRY C, vol. 123, no. 20, 23 May 2019 (2019-05-23), US, pages 12827 - 12840, XP055868593, ISSN: 1932-7447, DOI: 10.1021/acs.jpcc.9b02041
- CHUKANOV NIKITA V. ET AL: "Synthesis of Unsaturated Precursors for Parahydrogen-Induced Polarization and Molecular Imaging of 1- 13 C-Acetates and 1- 13 C-Pyruvates via Side Arm Hydrogenation", ACS OMEGA, vol. 3, no. 6, 30 June 2018 (2018-06-30), US, pages 6673 - 6682, XP055868578, ISSN: 2470-1343, DOI: 10.1021/acsomega.8b00983
- KORCHAK SERGEY ET AL: "Over 50?% 1 H and 13 C Polarization for Generating Hyperpolarized Metabolites-A para -Hydrogen Approach", vol. 7, no. 9, 13 July 2018 (2018-07-13), pages 672 - 676, XP055868763, ISSN: 2191-1363, Retrieved from the Internet <URL:https://onlinelibrary.wiley.com/doi/full-xml/10.1002/open.201800086> DOI: 10.1002/open.201800086
- KALTSCHNEE LUKAS ET AL: "Hyperpolarization of Amino Acids in Water Utilizing Parahydrogen on a Rhodium Nanocatalyst", vol. 25, no. 47, 26 July 2019 (2019-07-26), DE, pages 11031 - 11035, XP055868735, ISSN: 0947-6539, Retrieved from the Internet <URL:https://onlinelibrary.wiley.com/doi/full-xml/10.1002/chem.201902878> DOI: 10.1002/chem.201902878
- DE ET AL: "ETUDE STRUCTURALE D'ENOXYSILANES I. ATTRIBUTION DES SPECTRES DE VIBRATION DU TRIMETHYL- SILOXYETHYLENE, SES HOMOLOGUES DEUTERLES ET DES METHOXY- TRIMETHYLSILANES DIVERSEMENT DEUTERIES. DISCUSSION DES CONFORMATIONS MOLECULAIRES DE L'ENOXYSILANE", JOURNAL OF ORGANOMETALLIC CHEMISTY, vol. 81, 1 January 1971 (1971-01-01), pages 161 - 175, XP055902916
- JUNG M E ET AL: "Generation of the enolate of acetaldehyde from non-carbonyl substances and its C-alkylation, O-acylation and O-silylation", TETRAHEDRON LETTERS, ELSEVIER, AMSTERDAM , NL, vol. 18, no. 43, 1 January 1977 (1977-01-01), pages 3791 - 3794, XP026637041, ISSN: 0040-4039, [retrieved on 19770101], DOI: 10.1016/S0040-4039(01)83355-4
- LIMAT DOMINIQUE ET AL: "The Selective O-Acylation of Enolates Providing a Simple Entry to O-Enesters", TETRAHEDRON, vol. 51, no. 20, 1 January 1995 (1995-01-01), pages 5799 - 5806, XP055902911
- HOFECKER ANDREAS ET AL: "Novel synthesis routes for the preparation of low toxic vinyl ester and vinyl carbonate monomers", SYNTHETIC COMMUNICATIONS, vol. 50, no. 23, 22 September 2020 (2020-09-22), US, pages 3629 - 3641, XP055902904, ISSN: 0039-7911, DOI: 10.1080/00397911.2020.1808995
- STEEMERS LUUK ET AL: "Regio- and Stereoselective Chan-Lam-Evans Enol Esterification of Carboxylic Acids with Alkenylboroxines", ADVANCED SYNTHESIS AND CATALYSIS, vol. 360, no. 21, 26 September 2018 (2018-09-26), pages 4241 - 4245, XP093005996, ISSN: 1615-4150, Retrieved from the Internet <URL:https://onlinelibrary.wiley.com/doi/full-xml/10.1002/adsc.201800914> DOI: 10.1002/adsc.201800914

## Description

The present invention relates to a process for producing vinyl esters of carboxylic acids, especially vinyl esters of ketocarboxylic acids, which can be α- ketocarboxylic acids or β-ketocarboxylic acids. The vinyl esters of the carboxylic acids in their vinyl group can have hydrogen and have preferably deuterium in their vinyl group. The vinyl esters can be hydrogenated with *para*-hydrogen and the spin of the *para*-hydrogen can be transferred to the carbonyl carbon atom of the carboxyl group, which carbonyl-carbon atom is a ¹³C, followed by hydrolysis of the ester group, producing carboxylic acids, especially ketocarboxylic acids having a hyperpolarized ¹³C in the carbonyl carbon atom of the carboxyl group. Transfer of the spin order created by hydrogenation of the vinyl group with *para* hydrogen to achieve hyperpolarization of the ¹³C carbonyl atom of the carboxyl group can be accelerated by magnetic field cycling or by suitable NMR pulse sequences.

The process for producing vinyl esters of carboxylic acids, especially vinyl esters of ketocarboxylic acids, has the advantage of a high yield, of allowing the production of deuterated vinyl esters of ¹³C-carbonyl-labelled ketocarboxylic acids, and of ketocarboxylic acids having a hyperpolarized ¹³C as the carbonyl atom.

Products of the process are 1-¹³C-hyperpolarized carboxylic acids, preferably 1-¹³C-hyperpolarized ketocarboxylic acids that are e.g. suitable for use as magnetically labelled molecules in magnetic resonance imaging (MRI) diagnostics. A preferred ketocarboxylic acid ester is pyruvic acid vinyl ester, also called vinyl pyruvate, having a hyperpolarized ¹³C in the carbonyl carbon atom of the carboxyl group for use in diagnostic ¹³C-MRI.

### State of the art

EP 3 063 119 B1 describes producing 1-¹³C-hyperpolarized carboxylate containing compounds by providing an unsaturated ester of a carboxylate, hydrogenating the unsaturated ester with *para*-hydrogen to produce the *para*-hydrogenated ester, followed by spin order transfer to the 1-¹³C carbonyl carbon atom, e.g. by magnetic field cycling, which ester is converted by removal of the hydrogenated group to yield the 1-¹³C-hyperpolarized carboxylic acid. Particularly the allyl ester and the propargyl ester are suggested as substrates *for para-*hydrogenation because their synthesis from the corresponding alcohols and carboxylic acids is straightforward. Vinyl esters that are more efficient substrates for 1-¹³C-hyperpolarization (the spin ordered H atoms are closer to the ¹³C-carbonyl) are more difficult to prepare. A route for synthesis of vinyl pyruvate that could give acceptable yields is not described.

The unsaturated ester is kept in an organic solvent and reacted therein with para-hydrogen, then the magnetic field cycling is applied and the organic phase is mixed with an aqueous phase, which promotes hydrolysis of the ester and enrichment of the water-soluble 1-¹³C-hyperpolarized carboxylic acid in the aqueous phase.

Chukanov et al., ACS Omega 2018, 3, 6673-6682 describes a yield of 6% of synthesizing vinyl pyruvate from vinyl acetate and pyruvic acid with Pd^{II} acetate and KOH at 25°C for 3d, which was considered nonefficient and too low for spin transfer from para-hydrogen.

Salnikov et al., ChemPhysChem 2021, 22, 1389-1396, describes a synthetic procedure, related to Chukanov 2018, with slightly improved yields (8%), which also was not sufficient to perform hyperpolarization experiments. Salnikov et al., J. Phys. Chem C 2019, 12827-12840 describes the para-hydrogenation of the pyruvate vinyl ester having a natural ¹³C content.

Korchak et al., ChemistryOpen 2018 672-676 describes the para-hydrogenation of per-deuterated vinyl acetate with natural abundance of ¹³C to produce hyperpolarized acetate. As the precursor contained only the natural abundance of ¹³C, also the resulting hyperpolarized acetate can only contain the natural abundance of ¹³C of approx. 1.1%.

Kaltschnee et al., Chem. Eur. J. 2019, 11031-11035 relates to synthesis of deuterated vinyl esters of glycine and alanine, both of them amino acids with the carbonyl being ¹³C, using Boc-protection of the amino group in KOH with vinyl acetate-d₆, followed by deprotection in TFA. Hyperpolarization by para-hydrogenation with ESOTHERIC pulsed spin order transfer yielded polarizations of 0.8% for the ¹³C carbonyl of glycine and 0.65% for the ¹³C carbonyl of alanine.

J. van den Broeke, E. de Wolf, B.-J. Deelman, G. van Koten, Adv. Synth. Catal. 2003, 345, 625-635 describe a Rhodium-diphosphine catalyst with alkanes or cycloalkanes or alkane bridges as substituents at the phosphine ligands.

Steemers, L., L. Wijsman, J. H. van Maarseveen, Advanced Synthesis & Catalysis 2018) 360, 4241-4245 describe the preparation of vinyl esters of carboxylic acids.

W.-X. Lv., Q. Li, J.-L. Li, Z. Li, E Lin, D.-H. Tan, Y-H. Cai, W.-X. Fan, .H. Wang, Angew. Chem. Int. Ed. 2018, 57, 16544-16548, describe the production of vinyl boronic MIDA ester (MIDA=methyliminodiacetic acid).

Steemers et al. (Adv. Synth. Catal. 2018, 360, 4241-4245) describes a method to react deuterated vinyl boronic MIDA ester with arylcarboxylic acids.

### Object of the invention

It is an object of the invention to provide a process for efficiently producing a ketocarboxylic acid vinyl ester and its para-hydrogenation product, which after spin transfer and ester hydrolysis yields a hyperpolarized 1-¹³C carbonyl carbon compound, especially for use as a diagnostic compound in magnetic resonance imaging. Preferably, the process of the invention should produce the compound having a hyperpolarized 1-¹³C carbonyl carbon atom with high yield and at high purity. A further object is to provide a ketocarboxylic acid vinyl ester that allows for a higher efficiency of spin order transfer to the 1-¹³C carbonyl carbon.

### Description of the invention

The invention achieves the object by the features of the claims and especially provides a process in which a carboxylic acid halogenide, preferably chloride, or fluoride or bromide, of a ketocarboxylic acid is reacted with a trialkyl silylenolether for producing a ketocarboxylic acid vinyl ester and, as a by-product, a trialkyl silyl halogenide, accordingly a chloride, fluoride or bromide. The resulting ketocarboxylic acid vinyl ester can then be *para-*hydrogenated by hydrogenation with para-hydrogen, resulting in para-hydrogenation of the vinyl group. The polarisation transfer from the para-hydrogenated vinyl ester to the 1-¹³C carbonyl carbon of the ketocarboxylic acid can be accelerated by magnetic field cycling or by suitable NMR pulse sequences. Subsequent hydrolysis of the ester bond produces the free ketocarboxylic acid having a hyperpolarized 1-¹³C carbonyl carbon. The ketocarboxylic acid, and accordingly its carboxylic acid chloride, has a 1-¹³C carbonyl carbon. The trialkyl silylenolether is a trialkylsilyl vinylether.

The ketocarboxylic acid can be an alpha(α)-ketocarboxylic acid or a beta(β)-carboxylic acid.

In the following reaction schemes, the halogen atom is represented by the preferred chlorine. The carboxylic acid chloride can be produced by reacting a ketocarboxylic acid to a carboxylic acid chloride according to one of the following reaction schemes:

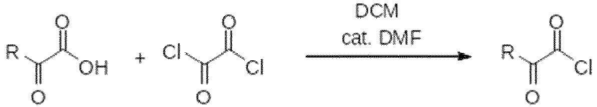

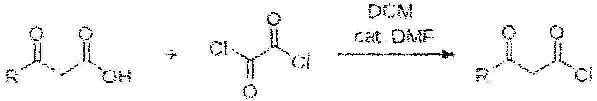

The ketocarboxylic acid chloride is produced from the corresponding ketocarboxylic acid by reacting it with a chlorinating agent, e.g. oxalyl chloride as shown here. As an alternative to the oxalyl chloride, thionyl chloride or phosphor-III-chloride or phosphor-V-chloride can be used. Preferred reagents are oxalic chloride and DMF as a catalyst, or 1-chloro-N,N,2-trimethylpropenylamine (tetramethyl-α-chlorenamine, TMCE) or any other suitable chlorinating reagent. The ketocarboxylic acid chloride in specific cases can be isolated, however, because of its instability the ketocarboxylic acid chloride is preferably further reacted in solution immediately after its production.

In a subsequent step, the α-, or β-ketocarboxylic acid halogenide, e.g. the α-, or β-ketocarboxylic acid chloride, is reacted with a trialkyl silylenolether to yield the corresponding vinylester. 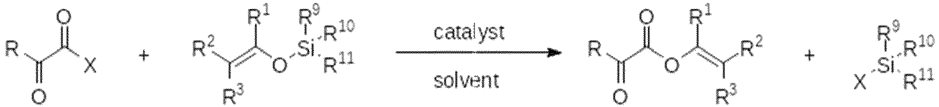 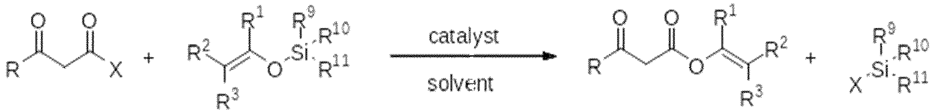 with X the halogen atom, preferably Cl, or Br or F. Upper reaction scheme for α-ketocarboxylic acid halogenide, lower reaction scheme for β-ketocarboxylic acid halogenide.

In the trialkyl silylenolether, R⁹, R¹⁰, R¹¹ can each independently be H or an alkyl, e.g. a C₁-to C₁₂- alkyl. R¹, R², R³ can each independently be H or an alkyl, e.g. a C₁- to C₁₂- alkyl, preferably each of R¹, R², R³ is D in order to produce a trialkyl silylenolether bound to a fully deuterated vinyl group.

Advantageously for hyperpolarization by subsequent para-hydrogenation and polarization transfer, the vinyl ester of the ketocarboxylic acid is deuterated at the vinyl group (all of R¹, R², R³ are D), and the carbonyl atom of the ketocarboxylic acid chloride is enriched in ¹³C.

In a preferred embodiment, the ketocarboxylic acid, and accordingly the acid chloride of the ketocarboxylic acid that is reacted with the trialkyl silylenolether, is fully deuterated, carrying deuterium instead of hydrogen atoms. A preferred deuterated ketocarboxylic acid is pyruvate in which the terminal methyl group is fully deuterated. Accordingly, in the preferred vinyl ester of the ketocarboxylic acid also the vinyl group is fully deuterated. A preferred vinyl ester of a ketocarboxylic acid that is obtainable by the process of the invention is of the structure 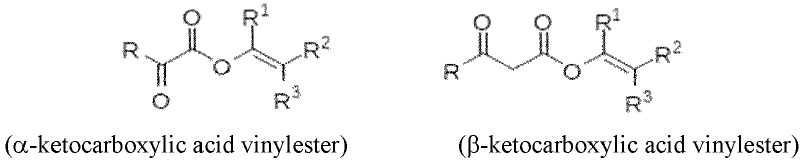 wherein R1, R2 and R3 are each deuterium, and R is a straight chain or branched C₁- to C₁₂-alkyl that preferably is fully deuterated, e.g. -CD₃, -CD₂-CD₃ , or -(CD₂)ₙ-CD₃ with n = 1, 2, 3 or 4, or R is a straight or branched C₁- to C₁₂-alkyl with a carboxyl or carbonylester at the end that preferably is fully deuterated, e.g. -(CD₂)ₙ-COOD, -(CD₂)ₙ-COO(CD=CD₂) and wherein the carboxyl carbon of the ketocarboxylic acid is ¹³C.

In embodiments in which the vinyl group of the ketocarboxylic acid is partially deuterated, preferably fully deuterated, the spin order that is introduced into the vinyl group by *para-*hydrogenation is more effectively transferred to the ¹³C atom of the carbonyl group. Deuteration of the R group in α-ketocarboxylic acid vinylesters leads to a longer half-life (*T*₁) of the hyperpolarized ¹³C.

The catalysts suitable for the reaction of acid chlorides with trialkyl silylenolethers are transition metal salts or transition metal complexes. Among the tested catalysts, PdCl₂ is preferred as it gave the best yields.

| Catalysts for the reaction of acid chlorides with trialkyl silylenolethers | | | |
|---|---|---|---|
| Catalyst | Eq. | Solvent | Yield^{[a]} |
| PdAc₂ | 0.04 | MeCN | 41 |
| PdCl₂ | 0.04 | MeCN | 80 |
| HgCl₂ | 0.04 | MeCN | 34 |

| | | | |
|---|---|---|---|
| [a] raw yield determined by ¹H-NMR after 20 h. | | | |

The systematic variation of solvents gave the following yields, showing that all solvents gave yields above 50%, with benzonitrile and dichloromethane being preferable, and with THF and acetonitrile most preferred.

| Entry | Kat. | Eq. | Solv. | Yield[%]^{[a]} |
|---|---|---|---|---|
| 1 | PdCl₂ | 0.04 | dichloromethane | 74 |
| 2 | PdCl₂ | 0.04 | THF | 80 |
| 3 | PdCl₂ | 0.04 | acetonitrile | 80 |
| 4 | PdCl₂ | 0.04 | benzonitrile | 56 |
| 5 | PdCl₂ | 0.04 | adiponitrile | 53 |
| 6 | PdCl₂ | 0.04 | toluene | 52 |
| 7 | PdCl₂ | 0.04 | 1,2 dimethoxyethane | 52 |

| | | | | |
|---|---|---|---|---|
| [a] raw yield determined by ¹H-NMR after 20 h reaction time. | | | | |

Preferably, the vinyl group of the trialkyl silylenolether is fully deuterated. Generally, a trialkyl silylenolether of a fully deuterated vinyl group can be produced by reacting fully deuterated tetrahydrofuran (THF), also termed deutero-oxolane-d₈, with butyllithium (BuLi) to give the lithium salt of the deuterated enolether of acetaldehyde, and further reacting the resulting product with a trialkylhalogensilane, e.g. trialkylchlorosilane, e.g. according to the following reaction scheme:
for R¹,R²,R³ = D 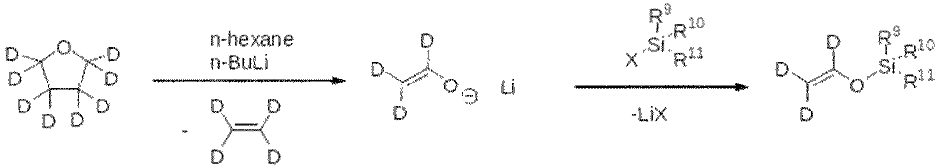
wherein X is a halogen atom, preferably chlorine.

The alkyl groups R⁹, R¹⁰, R¹¹ of the trialkylchlorosilane and accordingly of the trialkyl silylenolether of the deuterated vinyl can each be the same or can independently be selected from e.g. C₁- to C₁₂- alkyl, e.g. R9, R10 und R11 can each independently be branched alkyl, e.g. isopropyl or tert. butyl, or phenyl, e.g. R9, R10 are each methyl, R11 is iso-proypl or tert.-butyl; or R9, R10 are each methyl, R11 is phenyl.

It was found that in vinyl pyruvate, with the vinyl group carrying hydrogen, after *para-*hydrogenation and applying suitable NMR pulse sequences, approx. 15% of the spin order was transferred as hyperpolarization to the 1-¹³C carbonyl carbon of the ketocarboxylic acid group, whereas the same procedure applied to a deuterated vinyl group results in a transfer of approx. 96% hyperpolarization to the 1-¹³C carbonyl carbon.

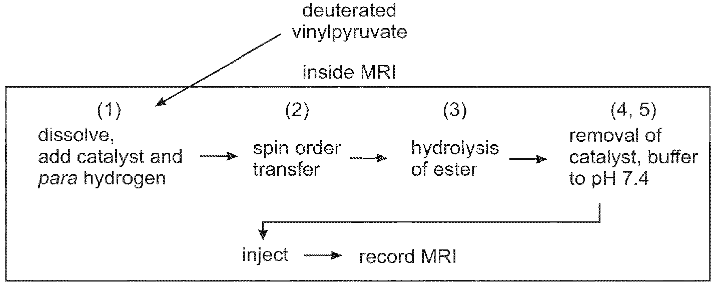

The ketocarboxylic acids either non-deuterated, preferably fully deuterated or deuterated in their vinyl groups, are stable and can be stored for extended periods of time. The following steps have to be performed in a magnetic field, preferentially inside a MRI scanner. Each step has to be performed as fast as possible because spin order and hyperpolarization decrease exponentially with relaxation.

In the process for producing a ketocarboxylic acid having a hyperpolarized carbonyl atom, the reaction mixture obtained by reacting the carboxylic acid halogenide of the ketocarboxylic acid with the trialkyl silylenolether, the vinyl ester of the ketocarboxylic acid ester that is isolated from the reaction mixture, optionally with a period of storing the reaction mixture, respectively the isolated vinyl ester of the ketocarboxylic acid ester, is contacted *with para-*hydrogen for *para*-hydrogenating the vinyl group of the ketocarboxylic acid ester with subsequent spin order transfer from the *para*-hydrogen atoms to hyperpolarize a ¹³C carbonyl carbon of the ketocarboxylic acid group, and the complete reaction mixture resulting from the para-hydrogenation is subjected to hydrolysis of the ester bond to produce the ketocarboxylic acid having a hyperpolarized 1-¹³C carbonyl carbon, and the then resulting complete reaction mixture is subsequently subjected to separation of the ketocarboxylic acid from the reaction mixture. The separated ketocarboxylic acid is then suitable for use in diagnostics, e.g. as a contrast medium for use in MRI diagnostics.
(1) In detail, before application in vivo, the vinyl ester of the ketocarboxylic acid is dissolved in water or in an organic solvent (e.g. chloroform, pentane) and treated with *para-*hydrogen under pressure and in the presence of a suitable hydrogenation catalyst. Thereby, the intrinsic spin order of *para*-hydrogen is transferred to the vinyl group, which is now an ethyl group.
(2) Magnetic field cycling or suitable NMR pulse sequences are now applied to transfer the spin order from the ethyl group into hyperpolarization of the ¹³C atom of the neighboring carbonyl group. This hyperpolarization transfer is much more efficient if the vinyl group is deuterated. Further deuteration of the alkyl group in the ketocarboxylic acid increases the half-life (*T*₁) of the hyperpolarized state, which is in the order of only 0.5-2 minutes.
(3) Hydrolysis of the ethylester with a strong base, e.g. aqueous NaOH, yields the free hyperpolarized ketocarboxylic acid salt, e.g. pyruvate, and ethanol. In the two-phase system, the latter two compounds are in the aqueous phase and the catalyst and unreacted starting compounds are in the organic (e.g. chloroform or pentane) phase.
(4) The strongly basic aqueous solution is brought to pH 7.4 by adding a buffer. Before injecting the hyperpolarized compound in vivo, the remaining traces of the hydrogenation catalyst have to be removed because these transition metal catalysts are toxic. A simple way to remove the catalysts is filtration over an ion exchange column. Any remaining traces of organic solvent also have to be removed, e.g. by passing the solution over a column of activated charcoal. This purification step can also be applied at an earlier stage.

The purified aqueous solution of the hyperpolarized ketocarboxylic acid is now ready for injection and MRI measurement.

This embodiment has the advantage of producing the ketocarboxylic acid having a hyperpolarized 1-¹³C carbonyl carbon in a short time while providing a high yield in substance and hyperpolarization and a high purity of the ketocarboxylic acid.

For para-hydrogenation, a hydrogenation catalyst is added to the reaction mixture.

Preferably, the catalyst is a homogeneous hydrogenation catalyst, preferentially a rhodium complex of the general formula [Rh(diphosphine)(diene)]⁺ such as [1,4-bis-(diphenylphosphino)-butane]-(1,5-cyclooctadiene)-rhodium(I)-tetrafluoroborate (79255-71-3) or (bicyclo[2.2.1]hepta-2,5-diene)-[1,4-bis-(diphenylphosphino)-butane]-rhodium(I)-tetrafluoroborate (82499-43-2). These hydrogenation catalysts are soluble in chloroform. When hydrolysing the para-hydrogenated vinyl ester by adding an alkaline aqueous solution to the organic chloroform phase, it was found that not only the desired acid moiety of the ester is transferred into the aqueous phase, but also an amount of the hydrogenation catalyst and of chloroform are transferred into the aqueous phase sufficiently high for a toxic effect in a recipient. For hydrogenation in water, the hydrogenation catalyst can be a watersoluble rhodium complex with the diphosphine ligand being 1,4-bis-[(phenyl-3-propansulfonate)-phosphine]-butane disodium salt

**In** an embodiment, the invention uses a process for para-hydrogenating a ketocarboxylic acid vinyl ester, preferably fully deuterated, with a para-hydrogenation catalyst that is soluble in a solvent consisting of an alkane, e.g. a C3- to C10-alkane, preferably a C4- to C8-alkane, which in each case can be an n-alkane or a cycloalkane, or a mixture of at least two of these, wherein preferably the catalyst is essentially soluble only in the solvent but insoluble in an aqueous alkaline solution.

Generally, the para-hydrogenation catalyst can be composed of a Rh⁺ ion bearing one diphosphine ligand or two monophosphine ligands, a diene ligand, e.g. 1,5-cyclooctadiene or norbornadiene, and an anion. Preferably, the solubility of the catalyst in solvents of low polarity is enhanced by using alkanes or cycloalkanes or alkane bridges as substituents at the phosphine ligands and in addition large and low-coordinating anions such as e.g. tetraarylboranes as anions.

Preferably, the para-hydrogenation is in the presence of a para-hydrogenation catalyst having the formula I

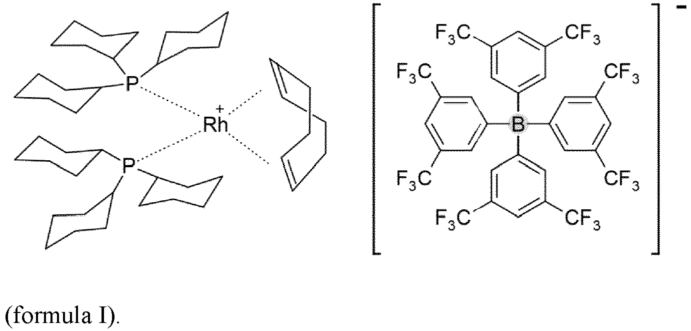

Preferably the catalyst is present in a solvent that consists of an alkane, liquid at the reaction temperature, e.g. a C3- to C10-alkane, preferably a C4- to C8-alkane, which in each case can be an n-alkane or a cycloalkane, or a mixture of at least two of these. A preferred alkane is pentane, e.g. n-pentane and/or iso-pentane, more preferred only n-pentane. The para-hydrogenation catalyst of formula I has the advantage of being soluble in an alkane, especially in pentane, but essentially insoluble in an aqueous phase, e.g. insoluble in an alkaline aqueous phase that contains the hyperpolarized alpha keto-acid that is the hydrolyzation product of the para-hydrogenated vinyl-ester of the ketocarboxylic acid, which alpha keto-acid preferably is pyruvic acid or its salt, preferably partially or fully deuterated. The catalyst of formula I has the advantage of not transferring from the solvent alkane phase into an adjacent aqueous phase, which effectively minimizes toxicity of the aqueous phase. The organic phase consisting of a C3- to C10-alkane, preferably a C4- to C8-alkane, preferably pentane, n-pentane and/or isopentane, has the advantage of essentially not migrating into the adjacent aqueous phase, and of having a low toxicity in a recipient human patient. Accordingly, for a fast production of the hyperpolarized ketocarboxylic acid or its salt in an aqueous phase, it is preferred to para-hydrogenate the vinyl ester of the ketocarboxylic acid in the presence of the catalyst of formula I in the solvent, to hyperpolarize the ¹³C of the carboxylic acid moiety, and to hydrolyse the resulting ester by adding an aqueous alkaline phase to the solvent, which results in the transfer of the hyperpolarized ketocarboxylic acid anion into the aqueous phase, with essentially no transfer of catalyst or solvent into the aqueous phase.

As an alternative to a solvent consisting of an alkane, the para-hydrogenation of the ketocarboxylic acid vinyl ester can be carried out in the presence of the catalyst, preferably the catalyst of formula **I,** in hypercritical carbon dioxide, e.g. under a carbon dioxide partial pressure of at least 30 bar. When para-hydrogenating the ketocarboxylic acid vinyl ester in hypercritical carbon dioxide, it is preferred, after the para-hydrogenation, to release the carbon dioxide and to subsequently add an alkaline aqueous solution for hydrolysis of the ester bond from the hyperpolarized ketocarboxylic acid. Therein, optionally, the para-hydrogenated ketocarboxylic acid vinyl ester after release of the carbon dioxide can be contacted with a solvent, e.g. an alkane, e.g. as described above, especially pentane, in order to dissolve the catalyst in pentane, and to then add an alkaline aqueous solution.

**In** the embodiment, in which the solvent consists of hypercritical carbon dioxide, it is preferred that subsequent to para-hydrogenation, the carbon dioxide is released, then an alkane solvent is added and thereafter an aqueous alkaline solution is added, resulting in hyperpolarized ketocarboxylic acid anion within the aqueous solution, while essentially retaining the catalyst within the alkane solvent.

The process of the invention has been found to give a yield of at least 60%, e.g. up to 85%, preferably up to 95%, for α-ketocarboxylic acids. This high yield is also obtained after the separation of the ketocarboxylic acid from the reaction mixture. It is currently believed that the high yield, and accordingly a high purity of the ketocarboxylic acid is caused by the steps of the reaction, especially caused by reacting a carboxylic acid chloride of a ketocarboxylic acid with a trialkyl silylenolether, and therefore minimizing the occurrence of reverse reactions or of dimerization, e.g. Aldol-reactions as they are currently assumed to cause the very low yield in the synthesis described by Chukanov et al., ACS Omega 2018. For the same reason the yields for β-ketocarboxylic acids according to our procedure are somewhat lower than those for α-ketocarboxylic acids.

The invention is now described in greater detail by way of examples.

### Example 1: Production of trimethylsiloxyethene-D3

An oven dried 100 mL flask flushed with nitrogen was charged with 27.9 mL (24.8 g, 344 mmol) of THF-D8, (fully deuterated tetrahydrofuran). The flask was cooled to 0°C and 7.82 mL (86.0 mmol) of 11 M n-butyllithium in n-hexane was added dropwise. After 0.5 h at 0°C the solution was warmed to room temperature and after additional 0.5 h heated to 40 °C. The solution was stirred at 40 °C for 16 h and the solvent was subsequently removed in vacuo. The remaining white solid was dissolved in 20 mL dry diglyme and further residues of THF where removed in vacuo (40°C, 50 mbar). An additional amount of 30 mL dry diglyme was added and the solution was cooled to 0°C. Then 11.2 mL (88.0 mmol) of dry trimethylsilyl chloride was slowly added and a significant amount of white precipitate was formed. After stirring for 2 h at room temperature the raw product was separated from the precipitate by flash distillation of the reaction mixture at 25°C and 50 mbar (reduced to 10 mbar during the process). The raw product containing some diglyme was then further distilled at atmospheric pressure and 75°C by Kugel Rohr distillation.

### Example 2: Production of dimethylisopropylsiloxyethene-D3

An oven dried 100 mL flask flushed with nitrogen was charged with 27.9 mL (24.8 g, 344 mmol) of THF-D8. The flask was cooled to 0°C and 7.82 mL (86.0 mmol) of 11 M *n-*butyllithium in n-hexane was added dropwise. After 0.5 h at 0°C the solution was warmed to room temperature and after additional 0.5 h heated to 40 °C. The solution was stirred at 40 °C for 16 h and the solvent was subsequently removed in vacuo. The residue was dissolved in 50 mL of dry diethylether and cooled to 0°C. Then 10.85 mL (9.47 g, 69.3 mmol) of dry dimethylisopropylsilyl chloride were slowly added to the solution and a significant amount of white precipitate was formed. After stirring for 2 h at room temperature the solution was transferred into a separatory funnel and washed with 50 mL saturated sodium bicarbonate solution once and two times with 50 mL of distilled water. The organic layer was dried over magnesium sulfate and the solvent was removed i. vac. The obtained raw product (>95 %) was used without further purification.

### Example 3: Production of vinyl pyruvate:

In a preferred embodiment to obtain the vinyl pyruvate, an oven dried 50 mL two neck flask flushed with nitrogen was charged with 10 mL dry dichloromethane. The flask was cooled to 0°C and 0.961 mL (1.58 g, 12.5 mmol) of oxalylchloride were added. 0.787 mL (1.00 g, 11.2 mmol) of pyruvic acid were mixed with 5 mL dried dichloromethane and 13 drops of N, N-dimethylformamide. The mixture was added dropwise to the cooled solution in the two-neck flask. The reaction was stirred for 2 h at 0°C and then another 2 h at room temperature. The solution was carefully degassed under Schlenk conditions. During the process, the volume of the solution was reduced from 15 mL to 10 mL (now referred to as solution A).

In an oven-dried 50 mL two neck flask flushed with nitrogen 80.0 mg (0.450 mmol) of palladium(II)chloride were placed, and 10 mL of dry dichloromethane were added. The flask was cooled to 0°C and 1.84 mL (1.43 g, 12.34 mmol) trimethyl(vinyloxy)silane as the silylenolether were added. The solution was stirred for 0.5 h at 0°C, then the solution A was added dropwise over a time of 0.5 h at 0°C. After complete addition, the solution was allowed to warm up to room temperature and was further stirred for 20 h. The solution was transferred into a one-neck flask, a spatula tip of hydroquinone (~30 mg, 0,27 mmol) was added and the solvent was removed at reduced pressure (600 mbar 30°C). The remains were collected in 3 mL dichloromethane: n-pentane (1:1) and then purified by flash column chromatography using a gradient of *n*-pentane, dichloromethane. The product was isolated as a slightly yellow oil in 68% yield.

### Example 4: Production of 1- ¹³C vinyl pyruvat-D6

In a preferred embodiment to obtain the ¹³C vinyl pyruvate-D6, an oven dried 50 mL two neck flask flushed with nitrogen was charged with 10 mL dry dichloromethane. The flask was cooled to 0° C and 0.961 mL (1.58 g, 12.5 mmol) of oxalylchloride were added. 0.787 mL (1.00 g, 11.2 mmol) of ¹³C pyruvic acid were mixed with 5 mL dried dichloromethane and 13 drops of N,N-dimethylformamide. The mixture was added dropwise to the cooled solution in the two-neck flask. The reaction was stirred for 2 h at 0° C and then another 2 h at room temperature. The solution was carefully degassed under Schlenk conditions. During the process, the volume of the solution was reduced from 15 mL to 10 mL (now referred to as solution A).

In an oven-dried 50 mL three neck flask flushed with nitrogen 80.0 mg (0.450 mmol) of palladium(II)chloride were placed, and 10 mL of dry dichloromethane were added. The flask was cooled to 0° C and 1.84 mL (1.82 g, 12.38 mmol) dimethylisopropyl(vinyloxy)silane-D3 as was added. The solution was stirred for 0.5 h at 0°C, then the solution A was added using a dropping funnel over a time of 0.5 h at 0° C. After complete addition, the solution was allowed to warm up to room temperature and was further stirred for 20 h. The solution was transferred into a one-neck flask, a spatula tip of hydroquinone (~30 mg, 0,27 mmol) was added and the solvent was removed at reduced pressure (600 mbar 30°C). The remains were collected in 3 mL dichloromethane: *n*-pentane (1:1) and then purified by flash column chromatography using a gradient of *n*-pentane, dichloromethane. The product was isolated as a slightly yellow oil in 29% yield.

### Example 5: Production of divinyl oxaloacetate (oxaloacetic acid divinyl ester))

To obtain the divinyl-oxaloacetate, α-oxaloacetic acid (1.00 g, 7.57 mmol) was placed in a three necked round bottom flask under N₂ atmosphere and brought into suspension with dichloromethane (75 ml). The flask was cooled to 0 °C and N,N-dimethylformamide (13 drops) was added. Afterwards oxalyl chloride (1.17 ml, 13.7 mmol) was added dropwise as a solution in dichloromethane (10 ml). The solution was stirred for 2 h at 0 °C and another 2 h at rt. The solution was degassed under Schlenk conditions and it's volume reduced to 35 ml to obtain a solution of α-oxaloacetic acid chloride (now referred to as solution A). Palladium(II)chloride (48.5 mg, 0.274 mmol) was placed in a round bottom flask under N₂ atmosphere and brought into suspension with dichloromethane (10 ml). The suspension was cooled to 0 °C and trimethyl vinyloxy silane (2.48 ml. 16.7 mmol) was added. The mixture was stirred for 0.5 h and the prior prepared solution A was added dropwise over 0.5 h. With no further cooling the solution was stirred for 20 h. The product was obtained after flash column chromatography using a gradient of n-pentane and dichloromethane with a yield of 17%. Optionally, the vinyl group of the trimethyl(vinyloxy)silane was deuterated.

### Example 6: Production of divinyl α-ketoglutarate (2-oxoglutarate divinylester)

To obtain the divinyl-α-ketoglutarate, α-ketoglutaric acid (1.00 g, 6.84 mmol) was placed in a three necked round bottom flask under N₂ atmosphere and brought into suspension with dichloromethane (75 ml). The flask was cooled to 0 °C and N,N-dimethylformamide (13 drops) was added. Afterwards oxalyl chloride (1.17 ml, 13.7 mmol) was added drop wise as a solution in dichloromethane (10 ml). The solution was stirred for 2 h at 0 °C and another 2 h at rt. The solution was degassed under Schlenk conditions and its volume reduced to 35 ml to obtain a solution of α-ketoglutaric acid chloride (now referred to as solution A). Palladium(II)chloride (48.5 mg, 0.274 mmol) was placed in a round bottom flask under N₂ atmosphere and brought into suspension with dichloromethane (10 ml). The suspension was cooled to 0 °C and trimethyl vinyloxy silane (2.48 ml. 16.7 mmol) was added. The mixture was stirred for 0.5 h and the prior prepared solution A was added dropwise over 0.5 h. With no further cooling the solution was stirred for 20 h. The product was obtained after flash column chromatography using a gradient of n-pentane and dichloromethane with a yield of 18%. Optionally, the vinyl group of the trimethyl(vinyloxy)silane was deuterated.

### Example 7: Production of vinyl acetoacetate (vinyl-3-oxobutyrate)

To obtain the vinyl acetoacetate, lithium acetoacetate (1.00 g, 9.26 mmol) was placed in a three necked round bottom flask under N₂ atmosphere and brought into suspension with dichloromethane (75 ml) and oxalic acid (anhydrous, 0.83 g, 9.26 mmol) and stirred for 5 min. The flask was cooled to 0 °C and DMF (13 drops) was added. Afterwards oxalyl dichloride (0.791 ml, 9.26 mmol) was added dropwise as a solution in dichloromethane (10 ml). The Solution was stirred for 2 h at 0° C and another 2 h at room temperature. The solution was degassed under Schlenk conditions and its volume reduced to 35 ml to obtain a solution of acetoacetic acid chloride, which cannot be isolated or stored because it rapidly decomposes under ambient conditions. Palladium(II)chloride (65.1 mg, 0.370 mmol) was placed in a round bottom flask under N₂ atmosphere and brought into suspension with dichloromethane (10 ml). The suspension was cooled to 0 °C and trimethyl vinyloxy silane (1.51 ml. 10.2 mmol) was added. The mixture was stirred for 0.5 h and the prior prepared solution of acetoacetic acid chloride was added drop wise over 0.5 h. With no further cooling the solution was stirred for 20 h. The product was obtained with a yield of 15 %. Optionally, the vinyl group of the trimethyl(vinyloxy)silane was deuterated.

***Para* hydrogenation:** The substrate precursors 1-¹³C-vinyl pyruvate-d₃ (or d₆) (6.8 mg) and hydrogenation catalyst [1,4-bis-(diphenylphosphino)-butan]-(1,5-cyclooctadien)-rhodium(I)-tetrafluoroborat (26 mg [Rh], CAS 79255-71-3) were dissolved in 6 mL of chloroform-d. ANMR tube (5 mm diameter, medium wall, high pressure) was filled with 450 µL of the above solution (vinyl pyruvate and Rh catalyst) and connected to a gas line. The gas line was flushed with N₂ for approximately 5 s at 5 bar before connecting to the NMR tube to prevent premature hydrogenation. The NMR tube was inserted into a NMR spectrometer (WB NMR 400 MHz, Avance Neo, 9.4 T, Bruker) with the probe set to 330 K. After 2-3 minutes waiting time to reach temperature stabilization, the pressure was built up by flushing *para* hydrogen through the sample for 10-15 seconds at 7 bar. Then the *para* hydrogen bubbling was stopped and a spin order transfer sequence was executed after 2 seconds.
The same procedure can be performed with other solvents including alkanes (e.g. pentane, butane, hexane etc), acetone, and alcohols and chlorine containing solvents. However, the hydrogenation is very inefficient in alkanes because of the low solubility of the catalyst in these solvents.

**Synthesis of a n-pentane soluble catalyst:** Bis-(1,5-cyclooctadien)-rhodium(I) - tetrafluoroborat (5 mg, 0.0123 mmol) and tricyclohexylphosphine (6.90 mg, 0.0246 mmol) and sodium tetrakis[3,5-bis(trifluoromethyl)phenyl]borate (10.9 mg, 0.0123 mmol) were dissolved in 1 ml dry DCM. To this clear orange solution *n*-pentane was slowly added until a white solid started to precipitate. The solid was filtered off and the solvent was evaporated under reduced pressure to obtain an orange solid. The orange solid was suspended in 10 ml of *n*-pentane. The supernatant yellow *n*-pentane solution was used for hyperpolarization experiments.

**Parahydrogenation in pentane:** 500 µl of the above pentane solution of the soluble catalyst was mixed with 10 µl of vinylpyruvate. Then the sample was placed into a 5 mm NMR tube with a low-pressure hydrogen gas line attached. The gas line was flushed with N₂ for approximately 5 s at 5 bar before connecting to the NMR tube. The NMR tube was inserted into a NMR spectrometer (WB NMR 400 MHz, Avance Neo, 9.4 T, Bruker) with the probe set to 330 K. After 2-3 minutes waiting time to reach temperature stabilization, the pressure was built up by flushing *para* hydrogen through the sample for 10-15 seconds at 7 bar. Then the *para* hydrogen bubbling was stopped and a spin order transfer sequence (PASADENA experiment) was executed after 2 seconds. Pentane lines were suppressed with OPSY (only *para* hydrogen spectroscopy) sequence.

**Spin order transfer:** The spin order transfer sequences: phINEPT+, Kadlecek, Goldman (10.1016/j.jmr.2012.08.016), SEPP-INEPT (10.1016/j.pnmrs.2012.03.001), ESOTHERIC (https://doi.org/10.1002/open.201800086) and their derivatives including adiabatic and shape RF pulses (10.1021/jz501754j) or variation of magnetic field (10.1021/acs.jpcb.5b06222) were used to transfer polarization from para-hydrogen to ¹³C. Experimentally we used ESOTHERIC SOT sequence that provides 100% polarization of ¹³C of 1-¹³C-ethyl pyruvate-d₆ after hydrogenation. Other ketocarboxylic acid esters provide similar degrees of hyperpolarization with the same procedure.

**Ester Hydrolysis:** To perform hydrolysis, the ¹³C-hyperpolarized ethyl ester (450 µL) is pushed out of the para-hydrogen reaction chamber into a glass vial that is prefilled with an aqueous solution of NaOH (450 µL,100 mmol/L). After pushing the hyperpolarized sample into the aqueous solution, the vial is shaken for about 3 seconds and let stand still to achieve phase separation for another 5-10 seconds. Then the top aqueous phase is collected. Traces of remaining catalyst are removed by pushing the aqueous phase through an ion exchange column and traces of solvent are removed by fast filtration through activated charcoal. If the ketocarboxylic ester is a pyruvic ester, the ester hydrolysis is performed in acetone-D6. Sodium pyruvate is scarcely soluble in acetone and is purified by precipitation, washing and re-dissolution in water.

The resulting hyperpolarized aqueous solution is filled into an NMR tube for quality analysis, or prefilled with enzymes or proteins for in vitro studies. If necessary, the pH of the solution is adjusted by addition of more acidic buffer to obtain the desired pH value (~7.4). After quality assurance (pH, purity, temperature) the hyperpolarized ¹³C- ketocarboxylic acid can be used for *in vivo* molecular MRI.

## Claims

1. Process for producing a ketocarboxylic acid vinyl ester by reacting a carboxylic acid halogenide of the ketocarboxylic acid with a trialkyl silylenolether, wherein the enolether moiety of the trialkyl silylenolether is a vinyl group.

2. Process according to claim 1, **characterized in that** the carboxylic acid halogenide of the ketocarboxylic acid is produced by reacting a ketocarboxylic acid with a halogenating agent.

3. Process according to one of the preceding claims, **characterized in that** halogenide is a chloride, a fluoride or a bromide.

4. Process according to one of the preceding claims, **characterized in that** the carboxyl carbon of the ketocarboxylic acid is ¹³C or a carbon having the natural or an enriched proportion of ¹³C.

5. Process according to one of the preceding claims, **characterized in that** the enolether moiety of the trialkyl silylenolether is fully deuterated.

6. Process according to one of the preceding claims, **characterized in that** the ketocarboxylic acid is fully deuterated.

7. Process according to one of the preceding claims, **characterized in that** the ketocarboxylic acid is pyruvate in which the terminal methyl group is fully deuterated.

8. Process according to claim 1, **characterized in that** the trialkyl silylenolether has a fully deuterated vinyl group and is produced by reacting fully deuterated tetrahydrofuran with butyllithium (BuLi), and reacting the reaction product with a trialkylhalogensilane.

9. Process according to one of the preceding claims, **characterized in that** the ketocarboxylic acid is an α-ketocarboxylic acid or a β-ketocarboxylic acid.

10. Process according to one of the preceding claims, **characterized in that** the vinyl group is *para*-hydrogenated with para-hydrogen, followed by spin order transfer from the *para*-hydrogenated vinyl group to a ¹³C carboxyl carbon of the ketocarboxylic acid, hydrolysis of the ester bond and separation of the resulting hyperpolarized ketocarboxylic acid from the reaction mixture.

11. Process according to one of the preceding claims, **characterized in that** during the *para*-hydrogenation the ketocarboxylic acid vinyl ester is present in a solvent that comprises or consists of at least one C₃- to C₁₀-alkane in the presence of a hydrogenation catalyst, and the *para*-hydrogen is introduced into the solvent.

12. Process according to one of claims 10 to 11, **characterized in that** during the *para-*hydrogenation the alkane is arranged adjacent to an alkaline aqueous phase, within in a pressure vessel.

13. Process according to one of claims 10 to 12, **characterized in that** the aqueous phase contains the ketocarboxylic acid or its anion and is separated from the alkane and from the hydrogenation catalyst by removing the aqueous phase from the alkane solvent phase.

14. Process according to one of the preceding claims, **characterized in that** the reaction mixture obtained by reacting the carboxylic acid halogenide of the ketocarboxylic acid with the trialkyl silylenolether is contacted with *para*-hydrogen *for para-*hydrogenating the vinyl group of the ketocarboxylic acid ester with subsequent spin transfer from the para-hydrogen atoms to hyperpolarize a ¹³C carboxyl carbon of the ketocarboxylic acid group, the resulting complete reaction mixture is subjected to hydrolysis of the ester bond to produce the ketocarboxylic acid having a hyperpolarized 1-¹³C carbonyl carbon, and the then resulting complete reaction mixture is subsequently subjected to separation of the ketocarboxylic acid.

15. Process according to one of the preceding claims, **characterized in that** during the *para*-hydrogenation the ketocarboxylic acid vinyl ester is present in a pressure vessel **in** a solvent that comprises or consists of hypercritical CO₂, in the presence of a hydrogenation catalyst, and the *para*-hydrogen is introduced into the solvent.

16. Process according to one of claims 10 to 15, **characterized in that** the *para-*hydrogenation catalyst is composed of a Rh⁺ ion bearing a diphosphine ligand or two monophosphine ligands, a diene ligand and an anion.

17. Process according to one of claims 15 to 16, **characterized in that** the hypercritical CO₂ solvent is removed by reducing the pressure within the pressure vessel, with subsequently contacting the residue with an alkaline aqueous phase, optionally in addition with a C₃- to C₁₀-alkane and subsequently separating the alkaline aqueous phase from the a C₃- to C₁₀-alkane.

18. Process according to one of the preceding claims, **characterized in that** the ketocarboxylic acid is separated from a reaction mixture by evaporating the solvent and the trialkyl halogen silane by-product and/or one chromatographic separation step.

19. Process according to one of claims 5 to 18, wherein producing deuterated trialkyl silylenolether is by reacting fully deuterated tetrahydrofuran (THF) with butyllithium (BuLi) and further reacting the resulting product with a trialkylhalogensilane, e.g. trialkylchlorosilane.

20. Vinyl ester of a ketocarboxylic acid having the structure: 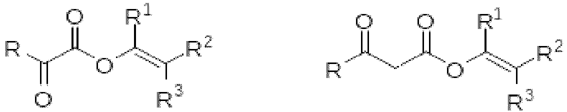 wherein R1, R2 and R3 are each deuterium, and R is a straight chain or branched C₁-**to** C₁₂-alkyl that is fully deuterated, and wherein the carboxyl carbon of the ketocarboxylic acid is ¹³C.

21. **Use** of a vinyl ester according to claim 20 for para-hydrogenation of the vinyl group with *para*-hydrogen, followed by spin order transfer from the para-hydrogenated vinyl group to the ¹³C carboxyl carbon of the ketocarboxylic acid, hydrolysis of the ester bond and separation of the resulting hyperpolarized ketocarboxylic acid from the reaction mixture.

## Patentansprüche

1. Verfahren zur Herstellung eines Ketocarbonsäurevinylesters durch Umsetzung eines Carbonsäurehalogenids der Ketocarbonsäure mit einem Trialkylsilylenolether, wobei der Enoletherrest des Trialkylsilylenolethers eine Vinylgruppe ist.

2. Verfahren nach Anspruch 1, **dadurch gekennzeichnet, dass** das Carbonsäurehalogenid der Ketocarbonsäure durch Umsetzung einer Ketocarbonsäure mit einem Halogenierungsmittel hergestellt wird.

3. Verfahren nach einem der vorhergehenden Ansprüche, **dadurch gekennzeichnet, dass** das Halogenid ein Chlorid, ein Fluorid oder ein Bromid ist.

4. Verfahren nach einem der vorhergehenden Ansprüche, **dadurch gekennzeichnet, dass** der Carboxylkohlenstoff der Ketocarbonsäure ¹³C oder ein Kohlenstoff mit dem natürlichen oder einem angereicherten Anteil an ¹³C ist.

5. Verfahren nach einem der vorhergehenden Ansprüche, **dadurch gekennzeichnet, dass** der Enoletheranteil des Trialkylsilylenolethers vollständig deuteriert ist.

6. Verfahren nach einem der vorhergehenden Ansprüche, **dadurch gekennzeichnet, dass** die Ketocarbonsäure vollständig deuteriert ist.

7. Verfahren nach einem der vorhergehenden Ansprüche, **dadurch gekennzeichnet, dass** die Ketocarbonsäure Pyruvat ist, bei dem die endständige Methylgruppe vollständig deuteriert ist.

8. Verfahren nach Anspruch 1, **dadurch gekennzeichnet, dass** der Trialkylsilylenolether eine vollständig deuterierte Vinylgruppe aufweist und durch Umsetzung von vollständig deuteriertem Tetrahydrofuran mit Butyllithium (BuLi) und Umsetzung des Reaktionsprodukts mit einem Trialkylhalogensilan hergestellt wird.

9. Verfahren nach einem der vorhergehenden Ansprüche, **dadurch gekennzeichnet, dass** die Ketocarbonsäure eine α-Ketocarbonsäure oder eine β-Ketocarbonsäure ist.

10. Verfahren nach einem der vorhergehenden Ansprüche, **dadurch gekennzeichnet, dass** die Vinylgruppe mit para-Wasserstoff *para*-hydriert wird, gefolgt von der Übertragung der Spin-Ordnung von der *para*-hydrierten Vinylgruppe auf einen ¹³C-Carboxylkohlenstoff der Ketocarbonsäure, Hydrolyse der Esterbindung und Abtrennung der resultierenden hyperpolarisierten Ketocarbonsäure aus dem Reaktionsgemisch.

11. Verfahren nach einem der vorhergehenden Ansprüche, **dadurch gekennzeichnet, dass** bei der *para*-Hydrierung der Ketocarbonsäurevinylester in einem Lösungsmittel, das mindestens ein C₃- bis C₁₀-Alkan enthält oder daraus besteht, in Gegenwart eines Hydrierungskatalysators vorliegt und der *para-*Wasserstoff in das Lösungsmittel eingebracht wird.

12. Verfahren nach einem der Ansprüche 10 bis 11, **dadurch gekennzeichnet, dass** das Alkan während der *para*-Hydrierung an eine alkalische wässrige Phase angrenzt, die sich in einem Druckbehälter befindet.

13. Verfahren nach einem der Ansprüche 10 bis 12, **dadurch gekennzeichnet, dass** die wässrige Phase die Ketocarbonsäure oder ihr Anion enthält und vom Alkan und vom Hydrierungskatalysator getrennt wird, indem die wässrige Phase aus der Alkan-Lösungsmittelphase entfernt wird.

14. Verfahren nach einem der vorhergehenden Ansprüche, **dadurch gekennzeichnet, dass** das durch Umsetzung des Carbonsäurehalogenids der Ketocarbonsäure mit dem Trialkylsilylenolether erhaltene Reaktionsgemisch mit *para*-Wasserstoff zur *para-*Hydrierung der Vinylgruppe des Ketocarbonsäureesters mit anschließendem Spin-Transfer von den *para*-Wasserstoffatomen zur Hyperpolarisierung eines ¹³C-Carboxylkohlenstoffs der Ketocarbonsäuregruppe in Kontakt gebracht wird, das resultierende vollständige Reaktionsgemisch der Hydrolyse der Esterbindung unterzogen wird, um die Ketocarbonsäure mit einem hyperpolarisierten 1-¹³C-Carbonylkohlenstoff herzustellen, und das dann resultierende vollständige Reaktionsgemisch anschließend der Abtrennung der Ketocarbonsäure unterzogen wird.

15. Verfahren nach einem der vorhergehenden Ansprüche, **dadurch gekennzeichnet, dass** bei der *para*-Hydrierung der Ketocarbonsäurevinylester in einem Druckbehälter in einem Lösungsmittel, das überkritisches CO₂ enthält oder daraus besteht, in Gegenwart eines Hydrierungskatalysators vorliegt und der *para*-Wasserstoff in das Lösungsmittel eingebracht wird.

16. Verfahren nach einem der Ansprüche 10 bis 15, **dadurch gekennzeichnet, dass** der *para*-Hydrierungskatalysator aus einem Rh⁺-Ion mit einem Diphosphinliganden oder zwei Monophosphinliganden, einem Dienliganden und einem Anion besteht.

17. Verfahren nach einem der Ansprüche 15 bis 16, **dadurch gekennzeichnet, dass** das überkritische CO₂-Lösungsmittel durch Druckminderung im Druckbehälter entfernt wird, der Rückstand anschließend mit einer alkalischen wässrigen Phase, gegebenenfalls zusätzlich mit einem C₃- bis C₁₀-Alkan, in Kontakt gebracht und anschließend die alkalische wässrige Phase von dem C₃- bis C₁₀-Alkan getrennt wird.

18. Verfahren nach einem der vorhergehenden Ansprüche, **dadurch gekennzeichnet, dass** die Ketocarbonsäure aus einem Reaktionsgemisch durch Verdampfen des Lösungsmittels und des Trialkylhalogensilan-Nebenprodukts und/oder einen chromatographischen Trennschritt abgetrennt wird.

19. Verfahren nach einem der Ansprüche 5 bis 18, wobei die Herstellung von deuteriertem Trialkylsilylenolether durch Umsetzung von vollständig deuteriertem Tetrahydrofuran (THF) mit Butyllithium (BuLi) und weitere Umsetzung des resultierenden Produkts mit einem Trialkylhalogensilan, z. B. Trialkylchlorsilan, erfolgt.

20. Vinylester einer Ketocarbonsäure mit der Struktur : 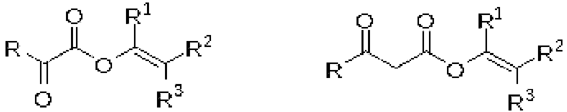 worin R1, R2 und R3 jeweils Deuterium sind und R ein geradkettiges oder verzweigtes C₁- bis C₁₂-Alkyl ist, das vollständig deuteriert ist, und worin der Carboxyl-Kohlenstoff der Ketocarbonsäure ¹³C ist.

21. Verwendung eines Vinylesters nach Anspruch 20 zur *para*-Hydrierung der Vinylgruppe mit *para*-Wasserstoff, gefolgt von einem Spin-Ordnungs-Transfer von der *para*-hydrierten Vinylgruppe zum ¹³C-Carboxylkohlenstoff der Ketocarbonsäure, Hydrolyse der Esterbindung und Abtrennung der resultierenden hyperpolarisierten Ketocarbonsäure aus dem Reaktionsgemisch.

## Revendications

1. Procédé de production d'un ester vinylique d'acide cétocarboxylique par réaction d'un halogénure d'acide carboxylique de l'acide cétocarboxylique avec un trialkyl silylénol éther, dans lequel le fragment énol éther du trialkylsilylénol éther est un groupe vinyle.

2. Procédé selon la revendication 1, **caractérisé en ce que** l'halogénure d'acide carboxylique de l'acide cétocarboxylique est produit par réaction d'un acide cétocarboxylique avec un agent halogénant.

3. Procédé selon l'une des revendications précédentes, **caractérisé en ce que** l'halogénure est un chlorure, un fluorure ou un bromure.

4. Procédé selon l'une des revendications précédentes, **caractérisé en ce que** le carbone carboxylique de l'e de l'acide cétocarboxylique est ¹³C ou un carbone ayant une proportion naturelle ou enrichie de ¹³C.

5. Procédé selon l'une des revendications précédentes, **caractérisé en ce que** le fragment énol-éther du trialkylsilylène-éther est entièrement deutéré.

6. Procédé selon l'une des revendications précédentes, **caractérisé en ce que** l'acide cétocarboxylique est entièrement deutéré.

7. Procédé selon l'une des revendications précédentes, **caractérisé en ce que** l'acide cétocarboxylique est le pyruvate dans lequel le groupe méthyle terminal est entièrement deutéré.

8. Procédé selon la revendication 1, **caractérisé en ce que** le trialkylsilylène éther a un groupe vinyle entièrement deutéré et est produit en faisant réagir du tétrahydrofurane entièrement deutéré avec du butyllithium (BuLi), puis en faisant réagir le produit de la réaction avec un trialkylhalogénosilane.

9. Procédé selon l'une des revendications précédentes, **caractérisé en ce que** l'acide cétocarboxylique est un acide α-cétocarboxylique ou un acide β-cétocarboxylique.

10. Procédé selon l'une des revendications précédentes, **caractérisé en ce que** le groupe vinyle est para-hydrogéné avec du para-hydrogène, suivi d'un transfert d'ordre de spin du groupe vinyle para-hydrogéné vers un carbone carboxyle ¹³C de l'acide cétocarboxylique, d'une hydrolyse de la liaison ester et d'une séparation de l'acide cétocarboxylique hyperpolarisé résultant du mélange réactionnel.

11. Procédé selon l'une des revendications précédentes, **caractérisé en ce que** pendant la para-hydrogénation, l'ester vinylique de l'acide cétocarboxylique est présent dans un solvant qui comprend ou consiste en au moins un C₃ à C₁₀ -alcane en présence d'un catalyseur d' hydrogénation, et le para-hydrogène est introduit dans le solvant.

12. Procédé selon l'une des revendications 10 à 11, **caractérisé en ce que**, pendant la para-hydrogénation, l'alcane est disposé à proximité d'une phase aqueuse alcaline, à l'intérieur d'un récipient sous pression.

13. Procédé selon l'une des revendications 10 à 12, **caractérisé en ce que** la phase aqueuse contient l'acide cétocarboxylique ou son anion et est séparée de l'alcane et du catalyseur d'hydrogénation par élimination de la phase aqueuse de la phase solvant alcane.

14. Procédé selon l'une des revendications précédentes, **caractérisé en ce que** le mélange réactionnel obtenu par réaction de l'halogénure d'acide carboxylique de l'acide cétocarboxylique avec le trialkylsilylénoléther est mis en contact avec du para-hydrogène pour para-hydrogéner le groupe vinyle de l'ester d'acide cétocarboxylique, suivi d'un transfert de spin à partir des atomes de para-hydrogène pour hyperpolariser un ¹³C du groupe acide cétocarboxylique, le mélange réactionnel complet résultant est soumis à une hydrolyse de la liaison ester pour produire l'acide cétocarboxylique ayant un carbone carbonyle 1-¹³C hyperpolarisé, et le mélange réactionnel complet résultant est ensuite soumis à une séparation de l'acide cétocarboxylique.

15. Procédé selon l'une des revendications précédentes, **caractérisé en ce que** pendant la para-hydrogénation, l'ester vinylique de l'acide cétocarboxylique est présent dans un récipient sous pression dans un solvant qui comprend ou consiste en CO₂ hypercritique, en présence d'un catalyseur d'hydrogénation, et le para-hydrogène est introduit dans le solvant.

16. Procédé selon l'une des revendications 10 à 15, **caractérisé en ce que** le catalyseur de para-hydrogénation est composé d'un ion Rh⁺ portant un ligand diphosphine ou deux ligands monophosphine, un ligand diène et un anion.

17. Procédé selon l'une des revendications 15 à 16, **caractérisé en ce que** le solvant CO₂ hypercritique est éliminé en réduisant la pression à l'intérieur du récipient sous pression, puis en mettant le résidu en contact avec une phase aqueuse alcaline, éventuellement en ajoutant un C₃ à C₁₀ -alcane, puis en séparant la phase aqueuse alcaline de l'alcane en C₃ à C₁₀ -alcane.

18. Procédé selon l'une des revendications précédentes, **caractérisé en ce que** l'acide cétocarboxylique est séparé d'un mélange réactionnel par évaporation du solvant et du sous-produit trialkylhalogénosilane et/ou par une étape de séparation chromatographique.

19. Procédé selon l'une des revendications 5 à 18, dans lequel la production d'éther de trialkylsilylène deutéré s'effectue en faisant réagir du tétrahydrofurane (THF) entièrement deutéré avec du butyllithium (BuLi) et en faisant ensuite réagir le produit résultant avec un trialkylhalogénosilane, par exemple un trialkylchlorosilane.

20. Esther vinylique d'un acide cétocarboxylique ayant la structure : 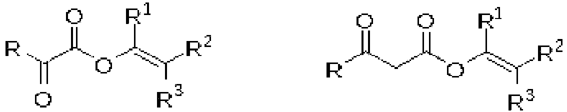
dans laquelle R1, R2 et R3 représentent chacun un deutérium, et R représente un groupe alkyle en C₁ à C₁₂ à chaîne droite ou ramifiée qui est entièrement deutéré, et
dans laquelle le carbone carboxyle de l'acide cétocarboxylique est ¹³C.

21. Utilisation d'un ester vinylique selon la revendication 20 pour la para-hydrogénation du groupe vinyle avec du para-hydrogène, suivie d'un transfert d'ordre de spin du groupe vinyle para-hydrogéné vers le carbone carboxyle ¹³C de l'acide cétocarboxylique, d'une hydrolyse de la liaison ester et d'une séparation de l'acide cétocarboxylique hyperpolarisé résultant du mélange réactionnel.
